# EUROPEAN PATENT APPLICATION

(11) **EP 3 939 596 A1**
(43) Date of publication of application: **19.01.2022**
(21) Application number: 20185806.5
(22) Date of filing: 14.07.2020
(51) Int. Cl.: A61K 31/704, A61K 36/25, A61P 33/10

(54) **A COMPOSITION FOR TREATING HELMINTH INFESTATION IN A MAMMAL**

(71) Applicant: Bimeda Animal Health Limited, D18 K8Y4 Dublin 18 (IE)
(72) Inventor: Smith, Brendan, Gerard, Dublin, D03V9F7 (IE); Gupta, Sandeep, Blessington, Co. Wicklow W91D2Y8 (IE); Lahmann, Martina, Bangor/Gwynedd, Wales LL57 2SZ (GB); Hoffmann, Karl, Aberystwyth, SY23 3DA (GB); Fisher, Maggie, Malvern, Worcestershire WR14 1UX (GB); Baird, Mark, Stephen, Porthmadog,Gwynedd, Wales LL49 9UY (GB)
(74) Representative: Purdylucey Intellectual Property

(57) **Abstract**

A composition for use in treating helminth infestation in a mammal is a described. The composition comprises an antihelmintically effective amount of α-hederin and a pharmaceutically acceptable carrier. The α-hederin is provided as an ivy extract. Treatment of liver fluke and rumen fluke infestation in ruminants is described.

## Description

### Field of the Invention

The present invention relates to a composition and compound for treating helminth infestation in a mammal, in particular treating *Fasciola hepatica* infestation.

### Background to the Invention

Infection by liver fluke (*Fasciola hepatica*) is estimated to cost the UK livestock industry over £300 million p.a., with a global cost of some US$3 billion. Pasture grazing livestock, primarily cattle, sheep, goats and to a lesser extent pigs and horses are all susceptible to the parasite.

Resistant strains of the liver fluke are now commonplace across the world, with not one of the currently available treatments unaffected.

To overcome the problem of resistance, some authorities have licensed the use of formulations comprising combinations of active ingredients, but there are no currently reported new compounds on the horizon.

α-hederin, a water soluble pentacyclic triterpenoid saponin found in certain species of plants including Nigella and Hedera, displays many biological activities including anti-inflammatory, anti-arthritic, anti-oxidant, cytotoxic and anti-tumour activity (Gepdiremen et a. 2005, Cheng et al. 2014, Islam 2017 and Adamska et al. 2019). α-hederin has also been found to exhibit anti-candida activity (Moulin-Traffort et al.) and antileishmanial activity in-vitro (Ridoux et al.).

It is an object of the invention to overcome at least any of the above-referenced problems.

### Summary of the Invention

The Applicant has addressed the problems of the prior art by identifying a natural compound present in ivy (Hedera) that is effective at treating helminth infestations in a mammal. The natural compound is α-hederin, a water soluble pentacyclic triterpenoid saponin. The compound is quite a large molecule and would not be anticipated to be absorbed from the rumen following oral administration, but would be expected to be absorbed from the small intestine. Surprisingly, the Cₘₐₓ for the compound following oral administration occurred after just four hours, indicating that rumen absorption of the intact molecule occurred. Data presented herein demonstrates that oral administration of α-hederin to sheep at an exemplary dosage of 50 mg per Kg animal body weight caused a 21.6% reduction in average fluke numbers compared with control sheep. This is highly surprising given the low plasma levels of α-hederin in treated sheep.

In a first aspect, the invention provides a composition for use in treating helminth infestation in a mammal, in which the composition comprises an antihelmintically effective amount of α-hederin and a pharmaceutically acceptable carrier.

In any embodiment, the α-hederin is isolated.

In any embodiment, the α-hederin is isolated from ivy.

In any embodiment, the α-hederin is an extract of ivy, in which the α-hederin typically constitutes at least 90% of the extract by weight. The ivy extract may be obtained by IMS (industrial methylated spirits) extraction of ivy material, for example leaves and/or stems, defatting using petroleum or an equivalent, and purification by silico column gel chromatography (in which the elution employs a chloroform:methanol:water mixture of 9:1:0.1 to 4:1:0.1). IMS extraction may involve maceration of the ivy material (a screw press juicer may be employed) to provide solids, pulp and juice. The solids and pulp may be mixed with IMS and incubated before solvent removal to provide crude saponin extract. More IMS may be added and the incubation and solvent removal steps repeated.

In any embodiment, the α-hederin is an extract of ivy.

In one embodiment, α-hederin constitutes at least 70%, 80%, 90%, 95%, 98% or 99% of the extract by weight.

In any embodiment, the composition is formulated for oral administration. The composition may be an oral suspension (e.g. an oral drench).

In any embodiment, the composition is administered at a dosage of 10-100 mg α-hederin per Kg animal weight.

In any embodiment, the composition is administered at a dosage of 30-70 mg α-hederin per Kg animal weight.

In any embodiment, the composition is administered at a dosage of 40-60 mg, or about 50 mg, α-hederin per Kg animal weight.

In any embodiment, the composition is formulated for subcutaneous administration.

In any embodiment, the composition is administered at a dosage of 5-10 mg α-hederin per Kg animal weight.

In any embodiment, the composition is a liquid and comprises 10 to 500 mg/ml, 50 to 500 mg/ml, 75 to 125 mg/ml or about 100 mg/ml α-hederin.

In any embodiment, the composition includes an additional active agent, for example another anthelmintic agent.

In any embodiment, the composition comprises an anthelmintic active agent that consists of α-hederin.

In another aspect, the invention provides a compound for use in treating helminth infestation in a mammal, in which the compound is α-hederin.

In any embodiment, the is α-hederin is obtained from ivy.

In any embodiment, the compound is orally administered.

In any embodiment the compound is administered at a dosage of 1-1000 mg α-hederin per Kg animal weight.

In any embodiment the compound is administered at a dosage of 1-100 mg α-hederin per Kg animal weight.

In any embodiment the compound is administered at a dosage of 30-70 mg α-hederin per Kg animal weight.

In any embodiment the compound is administered at a dosage of 40-60 mg or about 50 mg α-hederin per Kg animal weight.

In any embodiment, the compound is administered subcutaneously.

In any embodiment, the compound is subcutaneously administered at a dosage of 5-10 mg α-hederin per Kg animal weight.

In any embodiment, the helminth infestation is a Trematode infestation.

In any embodiment, the helminth infestation is a *Fasciola hepatica* infestation.

In any embodiment, the mammal is a ruminant, for example a bovine or ovine or caprine mammal.

In any embodiment, the composition is encapsulated.

In any embodiment, the α-hederin is encapsulated.

In any embodiment, the α-hederin is extracted from Hedera helix L (common ivy).

In another aspect, the invention provides a pharmaceutical composition comprising α-hederin in combination with a suitable pharmaceutical excipient.

In one embodiment, the α-hederin is provided in the form of an ivy extract, in which α-hederin constitutes at least 90, 95 or 99% of the ivy extract by weight.

In one embodiment, the ivy extract is an IMS extract of ivy.

In one embodiment, the ivy extract is purified by silica gel column chromatography.

In another aspect, the invention provides an ivy extract consisting essentially of α-hederin (e.g at least 95%, 98% or 99% α-hederin by weight).

In any embodiment, the α-hederin has a chemical formula of C₄₂H₆₈O₁₂.

In any embodiment, the α-hederin has a molecular weight of about 764 to 766 KDa.

Other aspects and preferred embodiments of the invention are defined and described in the other claims set out below.

### Brief Description of the Figures

FIG. 1: HPLC spectrum of crude ivy leaf.
FIG.2: HPLC spectrum of ivy extract purified by silica gel column chromatography

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to any or more of that entity. As such, the terms "a" (or "an"), "any or more," and "at least any" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, age, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an *effective amount* or a *therapeutically effective amount* or *antihelmintically effective amount* of an agent defines an amount that can be administered to a subject without excessive toxicity, irritation, allergic response, or other problem or complication, commensurate with a reasonable benefit/risk ratio, but any that is sufficient to provide the desired effect, e.g. a reduction in helminth numbers in the treated subject compared with an untreated subject. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure. Improvement may be observed in biological / molecular markers, clinical or observational improvements. In a preferred embodiment, the methods of the invention are applicable to humans, farm animals (cows, sheep, goats, deer), large racing animals (horses, camels, dogs), and domestic companion animals (cats and dogs).

In any embodiment, the dosage of α-hederin may be configured to achieve a plasma concentration (Cmax) of 10-1000, 50-500, 50-100, 100-150, 150-200, 250-300, 300-350, 350-400, 400-450, 450-500 ng/ml. The dosage may be configured to achieve the Cmax within 4-48 hours of administration. The dosage of α-hederin may be 1-1000, 1-500, 1-200, 1-100, 1-20, 20-40, 40-60, 60-80 or 80-100 mg/Kg. The dosage of α-hederin may be 5-20, 5-15, 8-12 or about 10 mg/Kg when the administration is parenteral or subcutaneous. The dosage of α-hederin may be 20-80, 30-70, 40-60 or about 50 mg/Kg when the administration is oral. The dosage may be administered once every day, or once every 2, 3, 4, 5, 6, 7 days. The dose may be administered every week, fortnight, month, two months, three months, or four months.

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, camels, bison, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human. As used herein, the term "equine" refers to mammals of the family *Equidae,* which includes horses, donkeys, asses, kiang and zebra.

As used herein the term "a-hederin" refers to a water soluble pentacyclic triterpenoid saponin that can be obtained from various plants including ivy (Hedera) and Nigella species. α-hederin from ivy is described in Phytomedicine 2005 Jun;12(6-7):440-4. The chemical structure of an α-hederin from common ivy (Hedera helix L) is provided below:

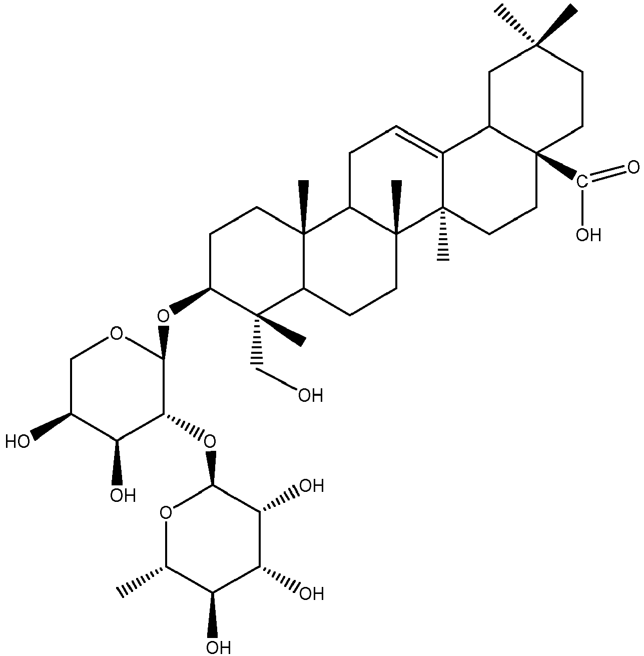

C₄₂H₆₈O₁₂ - MW 764.9 KDa.

As used herein the term "ivy" refers to Hedera species of plants, including *Hedera helix, Hedera poetarum, Hedera rhizomarifera, Hedera canariensis,* and *Hedera Hibernica.*

As used herein, the term "isolated" as applied to α-hederin means that the α-hederin is isolated from its natural environment, e.g. extracted from a plant (e.g. ivy) and generally provided as an extract in a concentrated form.

As used herein the term "helminth" refers to endoparasitic worms that include three main groups, Tapeworms (cestodes), Flukes (Trematodes) and Roundworms (Nematodes). Species of Flukes include *Shistosoma mansoni, Schistosoma japonicum, Fasciola hepatica (Liver fluke)* and *Paramphistomes* (Rumen fluke)

As used herein the term "ruminant" refers to a mammal that chews the cud regurgitated from its rumen. Examples include cattle, sheep, goats, antelopes, and deer.

As used here, the term "administration" refers to a conventional route of administration to an individual and covers oral or parenteral delivery in any suitable form, e.g., feed product, beverage, tablet, capsule, suspension, and solution. The term "parenteral" refers to subcutaneous, intracutaneous, intravenous, intramuscular, intraarticular, intraarterial, intrasynovial, intrasternal, intrathecal, intralesional, and intracranial injection, as well as various infusion techniques.

In some embodiments of the current invention, the composition or compound may be delivered via any one of liposomes, mixed liposomes, oleosomes, niosomes, ethosomes, millicapsules, capsules, macrocapsules, nanocapsules, nanostructured lipid carriers, sponges, cyclodextrins, vesicles, micelles, mixed micelles of surfactants, surfactant-phospholipid mixed micelles, millispheres, spheres, lipospheres, particles, nanospheres, nanoparticles, milliparticles, solid nanopartciles as well as microemulsions including water-in-oil microemulsions with an internal structure of reverse micelle and nanoemulsions microspheres, microparticles. In some embodiment, the compound may be conjugated to a to a binding partner. In one embodiment, the compound is modified with a reactive group configured to allow conjugation to the binding partner.

A variety of methods are available for preparing liposomes. See, e.g., Szoka et al., Ann. Rev. Biophys. Bioeng. 9:467 (1980), U.S. Pat. Nos. 4,186,183, 4,217,344, 4,235,871, 4,261,975, 4,485,054, 4,501,728, 4,774,085, 4,837,028, 4,235,871, 4,261,975, 4,485,054, 4,501,728, 4,774,085, 4,837,028, 4,946,787, PCT Publication No. WO 91/17424, Deamer & Bangham, Biochim. Biophys. Acta 443:629-634 (1976); Fraley, et al., PNAS 76:3348-3352 (1979); Hope et al., Biochim. Biophys. Acta 812:55-65 (1985); Mayer et al., Biochim. Biophys. Acta 858:161-168 (1986); Williams et al., PNAS 85:242-246 (1988); Liposomes (Ostro (ed.), 1983, Chapter 1); Hope et al., Chem. Phys. Lip. 40:89 (1986); Gregoriadis, Liposome Technology (1984) and Lasic, Liposomes: from Physics to Applications (1993)). Suitable methods include, for example, sonication, extrusion, high pressure/homogenization, microfluidization, detergent dialysis, calcium-induced fusion of small liposome vehicles and ether fusion methods, all of which are well known in the art.

These delivery systems may be adapted to achieve a greater penetration of the active compound. This may improve pharmacokinetic and pharmacodynamics properties. The delivery system may be a sustained release system wherein the compound is gradually released during a period of time and preferably with a constant release rate over a period of time. The delivery systems are prepared by methods known in the art. The amount of compound contained in the sustained release system will depend on where the composition is to be delivered and the duration of the release as well as the type of the condition, disease and/or disorder to be treated or cared for.

The compound of the invention may be administered by oral administration. The compound (and other ingredients, if desired) may also be enclosed in a hard or soft shell gelatin capsule, compressed into tablets, or incorporated directly into the subject's diet. For oral therapeutic administration, the compounds may be incorporated with excipients and used in the form of ingestible tablets, buccal tablets, troches, capsules, elixirs, suspensions, syrups, wafers, and the like. The compound may be coated, or co-administered with, a material to prevent its inactivation. The coating may be configured to protect the active agent during transit through the stomach and release the active agent in the ileum. In any embodiment, the composition is a liquid and comprises 10-500 mg/ml α-hederin.

The composition of the invention may include one or more pharmaceutically acceptable excipients. Examples of pharmaceutically acceptable excipients for the various different forms of pharmaceutical or veterinary compositions described herein may be found in the "Handbook of Pharmaceutical Excipients", 2nd Edition, (1994), edited by A Wade and PJ Weller. Acceptable carriers or diluents for therapeutic use are well known in the pharmaceutical art, and are described, for example, in Remington's Pharmaceutical Sciences, Mack Publishing Co. (A. R. Gennaro edit. 1985). Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavouring agents may be provided in the pharmaceutical composition. Examples of preservatives include sodium benzoate, sorbic acid and esters of phydroxybenzoic acid. Antioxidants and suspending agents may be also used.

The compound of the invention may be administered in the form of a conjugate of α-hederin conjugated, fused or linked to a binding partner, optionally via a linker molecule. Examples include one or more polyethylene glycol polymers or other compounds, such as molecular weight increasing compounds or lipophilic groups. The molecular weight increasing compound is any compound that will increase the molecular weight, typically by 10% to 90%, or 20% to 50% of the resulting conjugate and may have a molecular weight of between 200 and 20, 000, preferably between 500 and 10, 000. The molecular weight increasing compound may be PEG, any water-soluble (amphiphilic or hydrophilic) polymer moiety, homo or co-polymers of PEG, a monomethyl-subsitututed polymer of PEG (mPEG) and polyoxyethylene glycerol (POG), polyamino acids such as poly-lysine, poly-glutamic acid, poly-aspartic acid, particular those of L conformation, pharmacologically inactive proteins such as albumin, gelatin, a fatty acid, olysaccharide, a lipid amino acid and dextran. The polymer moiety may be straight chained or branched and it may have a molecular weight of 500 to 40000Da, 5000 to 10000 Da, 10000 to 5000, Da. The binding partner may be any suitable cell penetrating compound, such as tat peptide, penetratin, pep-1. The binding partner may be an antibody molecule. The binding partner may be a lipophilic moiety or a polymeric moiety. The lipophilic substituent and polymeric substituents are known in the art. The lipophilic substituent includes an acyl group, a sulphonyl group, an N atom, an O atom or an S atom which forms part of the ester, sulphonyl ester, thioester, amide or sulphonamide. The lipophilic moiety may include a hydrocarbon chain having 4 to 30 C atoms, preferably between 8 and 12 C atoms. It may be linear or branched, saturated or unsaturated. The hydrocarbon chain may be further substituted. It may be cycloalkane or heterocycloalkane. The binding partner may be conjugated via a spacer. The spacer may be a natural or unnatural amino acid, succinic acid, lysyl, glutamyl, asparagyl, glycyl, beta-alanyl, gamma-amino butanoyl. The binding partner may be conjugated via an ester, a sulphonyl ester, a thioester, an amide, a carbamate, a urea, a sulphonamide. A person skilled in the art is aware of suitable means to prepare the described conjugate.

The composition of the invention may include an additional active agent, for example an additional antihelmintic agent. Examples include Praziquantel, Clozantel, Halogenated salicylamides, Piperazine, Benzidimazoles, Morantel, Pyrantel, Levamisole, Emodepside and Ivermectin.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### Example 1 - Preparation of purified α-hederin

### Raw materials

Ivy leaves and small stems were harvested and processed using an industrial screw press juicer causing considerable increase in temperature and producing solids, pulp and juice. These were allowed to cool to room temperature over 18 h, then stored at -10°C. The juice contained mainly sugars which were discarded. A portions of solids (ILS) and pulp (ILP) was left in hot water (60°C) for 2 hours and then passed through a fine mesh and washed twice with hot water. The remaining feed on the mesh was dried and extracted.

### IMS extraction of Ivy leaf material

To 750g of ILS was added 5000mL of industrial methylated spirits (IMS) and 40 g of activated carbon in a 10 L flask. The mixture was stirred with a mechanical stirrer for five hours at 60°C and then left to stand overnight. The mixture was filtered under vacuum suction, then twice washed with 1 L of IMS. The solvent was abstracted in vacuo to obtain 126g (16.8%) of crude saponin extract. Reclaimed IMS (4000 mL) was added to the residue and second extraction was carried out for four hours at 60°C. After cooling down the mixture was filtered under vacuum suction, then washed with 1L of IMS. The solvent was abstracted in vacuo to obtain 55 g (7.3 %) of crude saponin extract. In total was obtained 181 g (24.1%) of crude saponin mixture.
The extraction of ILP was carried out using the same method described above yielding 9.5% of crude saponin.

### Defatting of crude extract of ILS

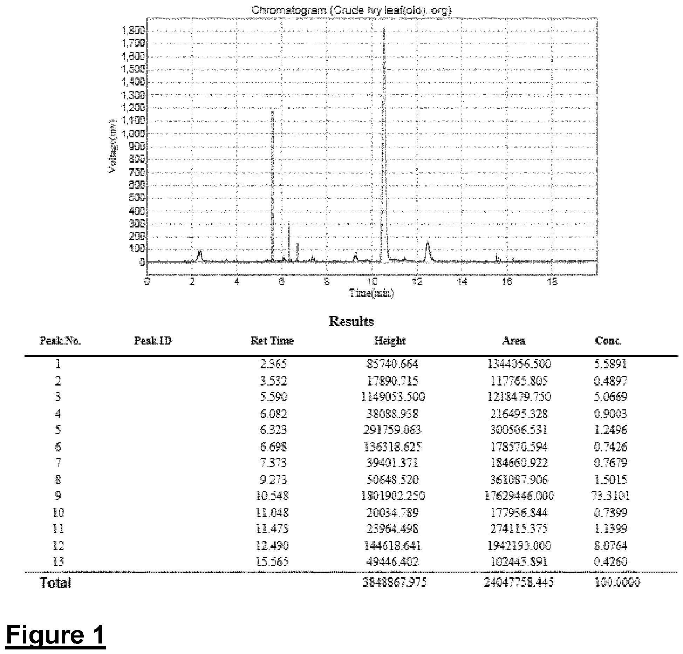

To 181g dried milled crude IMS extract of ILS was added 1000mL of petroleum in a 3L flask and the mixture was stirred with a mechanical stirrer for five hours at 40°C. The mixture was filtered under vacuum suction, then washed twice with 200mL of petroleum and dried gaining 171g (22.8%) of saponon extract.
The crude extracts of ILP and ILS were analysed by HPLC and found to contain a minimum of 71 - 80 % α-hederin accordingly (Fig. 1). This was by far the largest peak in the trace.

### Purification

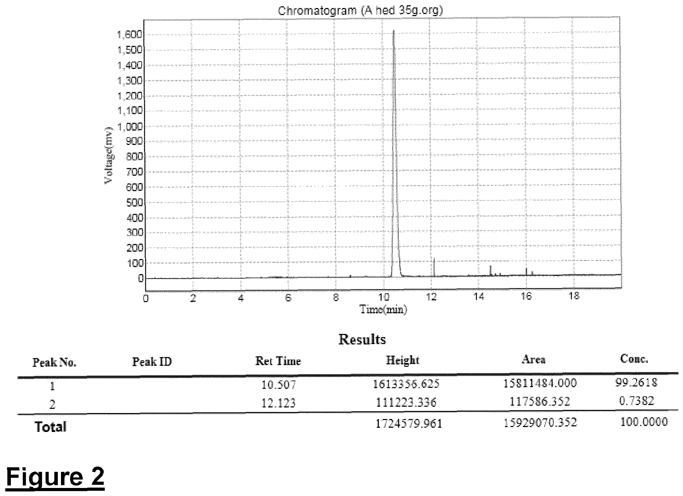

Purification of α-hederin was carried out by silica gel column chromatography eluted with Chloroform:Methanol:Water (9:1:0.1 to 4:1:0.1). The purified extract was found to contain more than 99% α-hederin (Fig. 2).

### Solubility and stability of α-hederin in 1,2-propanediol

- 5% α-hederin in 1,2-propanediol: soluble
- 10% α-hederin in 1,2-propanediol: soluble
After 13 days at room temperature the 10 % solution became cloudy, and the 5 % solution was cloudy at the top. At 35°C there were no visible changes over 3 weeks 10 wt.% α-hederin in 85.5 wt.% 1,2-Propanediol and 4.5 wt.% Benzyl alcohol was stable for 6 months at room temperature and at 5°C.

### Example 2 - In-vivo studies

IVL-11 is a relatively large particle and so would not be anticipated to be absorbed from the rumen following oral administration. Instead, absorption from the small intestine would be predicted. However, the Cmax after oral administration occurred after just four hours, suggesting that rumen absorption of the whole molecule has occurred as this time is far shorter than the transit time from oral administration to the small intestine (about 28 hours in cows (Mambrini and Payraud, 1997)).

Four sheep infected with adult fluke were treated with an IVL-11 oral solution formulation of Table 1:

**Table 1**

| | |
|---|---|
| Alpha hederin | 10 % wt |
| 1,2 propanediol | 85.5 % wt |
| Benzyl Alcohol | 4.5 % wt |

The solution was orally administered to the sheep to provide a dosage of α-hederin of 50 mg/kg and then worm counts compared to counts in untreated control sheep.

**Table 2: summary of efficacy following treatment of four sheep with IVL-11 orally at 50mg/kg compared with fluke counts in untreated control sheep**

| Compound and route administered | Dose-rate (mg/kg) | No. sheep | Arithmetic mean number of fluke at post mortem | % efficacy |
|---|---|---|---|---|
| IVL-11 ORAL | 50 | 4 | 83 | 21.6 |
| No treatment (control) | - | 2 | 106 | - |

IVL-11 treated sheep had a 21.6% reduction in average fluke numbers compared to the control sheep. Whilst the number of sheep was small there is a reduction in fluke numbers, something that is remarkable given that the low blood levels of IVL-11 following administration in vivo indicated that treatment would have no effect on fluke survival.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

### References

Adamska et al. Molecules (2019), 24(16), 2958.
Cheng et al. (2104) International Journal of Oncology 45(2) 757-763.
Gepdiremen et al. Phytomedicine 12 (2005) 440-444.
Mambrini, M and Payraud, JL, 1997. Reprod Nut Dev 37 427-442

## Claims

1. A composition for use in treating helminth infestation in a mammal, in which the composition comprises an antihelmintically effective amount of α-hederin and a pharmaceutically acceptable carrier.

2. A composition of Claim 1 for use of Claim 1, in which the α-hederin is obtained from ivy.

3. A composition of Claim 1 or 2 for use of Claim 1, in which the helminth infestation is a Trematode infestation.

4. A composition of Claim 1 or 2 for use of Claim 3, in which the helminth infestation is a *Fasciola hepatica* (Liver fluke) or *Paramphistomes* (Rumen fluke) infestation.

5. A composition of Claim 1 or 2 for use of Claim 1, 3 or 4, in which the composition is administered orally.

6. A composition of Claim 1 or 2 for use of Claim 5, in which the composition is administered at a dose of 30-70 mg α-hederin per Kg animal weight.

7. A composition of Claim 1 or 2 for use of Claim 1, 3 or 4, in which the composition is administered subcutaneously.

8. A composition of Claim 1 or 2 for use of Claim 7, in which the composition is administered at a dose of 5-10 mg α-hederin per Kg animal weight.

9. A composition of Claim 1 or 2 for use of any of Claim 1 and 3 to 7, in which the mammal is a ruminant.

10. A composition of Claim 2 for use of any of Claim 1 and 3 to 8, in which the composition comprises an extract of ivy leaf, in which α-hederin constitutes at least 90% of the extract by weight.

11. A pharmaceutical composition comprising α-hederin and a pharmaceutically acceptable carrier, in which the α-hederin is provided as an ivy extract containing at least 90% α-hederin by weight.

12. A pharmaceutical composition according to Claim 11 in which the ivy extract contains at least 98% α-hederin by weight.

13. A pharmaceutical composition according to Claim 11 or 12 formulated for oral administration to a ruminant and comprising 10 to 500 mg/ml α-hederin.

14. A pharmaceutical composition according to Claim 13 in the form of an oral suspension.
